# EUROPÄISCHE PATENTSCHRIFT

(11) **EP 2 560 039 B1**
(45) Veröffentlichungstag und Bekanntmachung des Hinweises auf die Patenterteilung: **28.12.2016**
(21) Anmeldenummer: 12176340.3
(22) Anmeldetag: 13.07.2012
(51) Int. Cl.: G02B 23/24, A61B 1/00, A61B 1/06

(54) **Endoskop und Verfahren zum Fertigen eines distalen Endes eines Endoskops**
Endoscope and method for producing a distal end of an endoscope
Endoscope et procédé destiné à la fabrication de l'extrémité distale d'un endoscope

(30) Priorität: 17.08.2011 DE 102011081067
(43) Veröffentlichungstag der Anmeldung: 20.02.2013
(73) Patentinhaber: Karl Storz GmbH & Co. KG, 78532 Tuttlingen (DE)
(72) Erfinder: Bürk, André, 78056 Villingen-Schwenningen (DE); Eisenkolb, Peter, 78532 Tuttlingen (DE)

(56) Entgegenhaltungen:
- EP-A1- 2 263 518
- US-A1- 2004 236 183
- US-A1- 2010 063 479

## Beschreibung

Die vorliegende Erfindung bezieht sich auf ein Endoskop, insbesondere das distale Ende eines Außenschafts eines Endoskops, und auf ein Verfahren zum Fertigen eines distalen Endes eines Außenschafts für ein Endoskop.

Viele Endoskope weisen eine Blickrichtung parallel zur Längsache ihres Schafts auf. In zunehmenden Maße werden jedoch Endoskope mit einer anderen feststehenden oder mit einstellbarer Blickrichtung nachgefragt und entwickelt. Ferner werden Endoskope mit einstellbarem Bildwinkel (field of view) bzw. einstellbarer Brennweite und weiteren Zusatzfunktionen nachgefragt und entwickelt. Zur Erschließung neuer Einsatzbereiche wird der Schaftdurchmesser immer weiter reduziert. Dabei trotzdem den für Einrichtungen des Beobachtungsstrahlengangs, des Beleuchtungsstrahlengangs, zum Einstellen der Blickrichtung, des Bildwinkels oder für andere Funktionen erforderlichen Bauraum bereitzustellen, stellt eine große Herausforderung dar.

In EP 2 263 518 A1 ist ein Endoskop beschrieben, bei dem das "Frontbauteil" bzw. der Abschlussabschnitt des Mantels des Endoskops aus dem Vollen gefräst wird.

In US 2004/0236183 A1 ist ein Arthroskop mit veränderbarer Blickrichtung beschrieben. In einem Außenrohr ist eine Baugruppe und ein Teil einer Lichtübertragungsanordnung eingebracht.

Eine Aufgabe der vorliegenden Erfindung besteht darin, ein verbessertes Endoskop und ein verbessertes Verfahren zum Fertigen eines distalen Endes eines Außenschafts für ein Endoskop zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, ein distales Ende eines Außenschafts für ein Endoskop aus zwei flächigen Bauteilen zusammenzusetzen, nämlich einem zylindermantelförmigen Mantelbauteil und einem Frontbauteil, das insbesondere die Gestalt einer lediglich in einer Richtung gekrümmten Fläche aufweist.

Ein Endoskop umfasst einen Außenschaft mit einem distalen Ende, dessen Oberfläche durch eine Mantelfläche und eine gekrümmte Frontfläche gebildet wird, wobei das distale Ende des Außenschafts ein Mantelbauteil und ein gekrümmtes Frontbauteil, das an das Mantelbauteil gefügt ist, umfasst, wobei ein Abstand einer Fügefläche zwischen dem Mantelbauteil und dem Frontbauteil von einer Kante zwischen der Mantelfläche und der gekrümmten Frontfläche nicht größer als eine Wandstärke des Mantelbauteils oder nicht größer als eine Wandstärke des Frontbauteils ist.

Das distale Ende des Außenschafts kann als separat gefertigtes und dann an ein Schaftrohr gefügtes Bauteil oder von vornherein einstückig mit dem Schaftrohr des Endoskops vorgesehen sein. Die Mantelfläche des distalen Endes des Außenschafts weist insbesondere die Gestalt eines Zylindermantels mit kreisförmigem oder anderem Querschnitt auf, wobei alle Flächennormalen der Mantelfläche senkrecht zur Längsachse des Endoskops sind. Die Frontfläche bzw. Stirnfläche ist insbesondere parallel zu einer vorbestimmten Richtung senkrecht zur Längsachse des Endoskops. Insbesondere weist die Frontfläche bzw. Stirnfläche die Gestalt eines Ausschnitts einer Mantelfläche eines Zylinders mit kreisförmigem oder nicht-kreisförmigem Querschnitt und Symmetrieachse senkrecht zur Längsachse des Endoskops auf.

Soweit die Kante zwischen Mantelfläche und Frontfläche gebrochen oder abgerundet ist oder eine Fase aufweist, ist mit dem Abstand zu dieser Kante der Abstand zu der Linie gemeint, in der sich Mantelfläche und Frontfläche bei nicht gebrochener oder nicht abgerundeter Kante bzw. vor dem Brechen oder Abrunden der Kante schnitten.

Sowohl das Mantelbauteil als auch das Frontbauteil weisen jeweils insbesondere eine konstante oder im Wesentlichen konstante Wandstärke auf, wie dies beispielsweise für Bauteile aus gebogenem bzw. plastisch verformtem Blech in der Regel gilt.

Ein Abstand der Fügefläche zwischen Mantelbauteil und Frontbauteil von der Kante zwischen Mantelfläche und Frontfläche, der nicht größer als die Wandstärke des Mantelbauteils und/oder nicht größer als die Wandstärke des Frontbauteils ist, ist insbesondere mit nachfolgend beschriebenen Merkmalen und Fertigungsverfahren realisierbar, die es ermöglichen, im Inneren des distalen Endes des Außenschafts besonders viel Bauraum bereitzustellen. Ferner kann insbesondere die Fertigung des Frontbauteils mit vergleichsweise geringem Aufwand und somit kostengünstig möglich sein.

Bei einem Endoskop, wie es hier beschrieben ist, bildet das Mantelbauteil höchstens von einem Randstreifen, dessen Breite durch die Wandstärke des Frontbauteils bedingt ist, abgesehen die gesamte Mantelfläche des distalen Endes des Schafts.

Somit wird die Mantelfläche des distalen Endes des Außenschafts vollständig durch einen entsprechenden Oberflächenbereich des Mantelbauteils gebildet, oder ein an die Kante zwischen Mantelfläche und Frontfläche angrenzender Randstreifen, dessen Breite der Wandstärke des Frontbauteils geteilt durch den Sinus des Winkels zwischen Mantelfläche und Frontfläche entspricht, wird von dem Frontbauteil gebildet, und der Rest der Mantelfläche von dem Mantelbauteil.

Bei einem Endoskop, wie es hier beschrieben ist, kann das Frontbauteil von Öffnungen im Frontbauteil abgesehen die gesamte Frontfläche des distalen Endes des Außenschafts bilden.

Öffnungen im Frontbauteil sind insbesondere eine Öffnung für ein Fensterbauteil im Beobachtungsstrahlengang und eine oder mehrere Öffnungen für Lichtaustrittsflächen im Beleuchtungsstrahlengang des Endoskops.

Bei einem Endoskop, wie es hier beschrieben ist, kann das Frontbauteil von Öffnungen im Frontbauteil und von einem Randstreifen, dessen Breite durch die Wandstärke des Mantelbauteils bedingt ist, abgesehen die gesamte Frontfläche des distalen Endes des Außenschafts bilden.

Somit wird die Frontfläche des distalen Endes des Außenschafts von Öffnungen im Frontbauteil abgesehen vollständig durch einen entsprechenden Oberflächenbereich des Frontbauteils gebildet, oder ein an die Kante zwischen Mantelfläche und Frontfläche angrenzender Randstreifen, dessen Breite der Wandstärke des Mantelbauteils geteilt durch den Sinus des Winkels zwischen Mantelfläche und Frontfläche entspricht, wird von dem Mantelbauteil gebildet, und der Rest der Mantelfläche von dem Frontbauteil.

Wie insbesondere anhand der nachfolgend beschriebenen Beispiele deutlich wird, kann ein Frontbauteil, das von einem Randstreifen, dessen Breite durch die Wandstärke des Mantelbauteils bedingt ist, abgesehen die gesamte Frontfläche des distalen Endes des Außenschafts bildet, hinsichtlich des Fügens des Frontbauteils an das Mantelbauteil Vorteile aufweisen. Ein Frontbauteil, das die gesamte Frontfläche des distalen Endes des Außenschafts bildet, kann besonders viel Fläche für Öffnungen im Frontbauteil bereitstellen.

Bei einem Endoskop, wie es hier beschrieben ist, bilden das Frontbauteil eine Teilfläche der Mantelfläche und das Mantelbauteil eine Teilfläche der Frontfläche des distalen Endes des Außenschafts.

Insbesondere wird die Kante zwischen Mantelfläche und Frontfläche teilweise vom Mantelbauteil und teilweise vom Frontbauteil gebildet. In dem Bereich oder in den Bereichen, in denen das Mantelbauteil die Kante bildet, reicht das Mantelbauteil in die Frontfläche hinein, insbesondere in Form eines schmalen Streifens, dessen Breite durch die Wandstärke des Mantelbauteils und den Winkel zwischen Mantelfläche und Frontfläche bestimmt ist. In dem Bereich oder in den Bereichen, in denen das Frontbauteil die Kante bildet, reicht das Frontbauteil in die Mantelfläche hinein, insbesondere in Form eines schmalen Streifens, dessen Breite durch die Wandstärke des Frontbauteils und den Winkel zwischen Mantelfläche und Frontfläche bestimmt ist.

Die Grenzen zwischen aneinandergrenzenden Bereichen, in denen abwechselnd das Mantelbauteil und das Frontbauteil die Kante zwischen Mantelfläche und Frontfläche bilden, ermöglichen eine präzise formschlüssige Ausrichtung des Frontbauteils relativ zum Mantelbauteil. Anders ausgedrückt verhindern diese Grenzen vor dem Fügen oder während des Fügevorgangs eine Verschiebung des Frontbauteils relativ zum Mantelbauteil parallel zur Kante zwischen Mantelfläche und Frontfläche. Dies kann die Fertigung des distalen Endes vereinfachen und damit die Fertigungskosten verringern. Ferner kann die erreichbare Präzision erhöht werden, was gerade angesichts zunehmender Miniaturisierung von Bedeutung ist.

Bei einem Endoskop, wie es hier beschrieben ist, weist das Frontbauteil insbesondere die Gestalt einer Fläche mit gaußscher Krümmung K = 0 auf.

Ein Frontbauteil mit der Gestalt einer Fläche mit verschwindender gaußscher Krümmung kann eine besonders einfache Fertigung ermöglichen, beispielsweise durch Biegen eines Blechs in lediglich einer Richtung oder durch Drahterosion. Ferner ist eine Fertigung mittels eines zylindrischen Fräs- oder Schleifwerkzeugs, das lediglich entlang einer einzigen Kurve in einer Ebene senkrecht zu seiner Rotationsachse bewegt werden muss, möglich.

Bei einem Endoskop, wie es hier beschrieben ist, ist das Frontbauteil insbesondere aus einem plastisch verformten Blech gebildet.

Die Bildung des Frontbauteils aus einem in mehreren Richtungen gekrümmten Blech (gaußsche Krümmung K ≠ 0) und insbesondere die Bildung des Frontbauteils aus einem in einer Richtung gekrümmten Blech (verschwindende gaußsche Krümmung K = 0) kann mit der erforderlichen Präzision und gleichzeitig kostengünstig erfolgen. Ferner ist mit einem plastisch verformten Blech eine geringe Wandstärke realisierbar, so dass im Innern des distalen Endes besonders viel nutzbarer Bauraum verbleibt.

Bei einem Verfahren zum Fertigen eines distalen Endes eines Außenschafts für ein Endoskop, bei dem die Oberfläche des distalen Endes des Außenschafts durch eine Mantelfläche und eine gekrümmte Frontfläche gebildet wird, die in einer Kante aneinandergrenzen, werden ein Mantelbauteil bereitgestellt, ein gekrümmtes Frontbauteil bereitgestellt und das Frontbauteil an einer Fügefläche, deren Abstand von der Kante zwischen Mantelfläche und Frontfläche nicht größer als eine Wandstärke des Mantelbauteils oder nicht größer als eine Wandstärke des Frontbauteils ist, an das Mantelbauteil gefügt.

Die Mantelfläche des distalen Endes des Außenschafts weist insbesondere die Gestalt eines Zylindermantels mit kreisförmigem oder nicht-kreisförmigem Querschnitt auf, wobei die Flächennormalen der Mantelfläche senkrecht zur Längsachse des Endoskops, zumindest senkrecht zur Längsachse des distalen Endes des Außenschafts des Endoskops sind. Die Flächennormalen der gekrümmten Frontfläche weisen andere Orientierungen auf. Insbesondere sind alle Abschnitte der Frontfläche parallel zu einer vorbestimmten Richtung senkrecht zur Längsachse des Endoskops oder des distalen Endes des Außenschafts des Endoskops. Insbesondere weist die Frontfläche die Gestalt eines Ausschnitts eines Zylindermantels mit kreisförmigem oder nicht-kreisförmigem Querschnitt und Symmetrieachse senkrecht zur Längsachse des Endoskops oder des distalen Endes des Außenschafts auf.

Soweit die Kante zwischen Mantelfläche und Frontfläche gebrochen oder abgerundet ist, ist mit dem Abstand zu dieser Kante der Abstand zu der Linie gemeint, in der sich Mantelfläche und Frontfläche bei einer nicht gebrochenen oder nicht abgerundeten Kante schnitten.

Bei einem Verfahren, wie es hier beschrieben ist, kann das Bereitstellen des Frontbauteils ein Bereitstellen eines Werkstücks und ein Erzeugen des Frontbauteils aus dem Werkstück zumindest entweder mittels Erodierens oder mittels Laserschneidens umfassen.

Das Werkstück ist insbesondere ein Vollzylinder mit einem Querschnitt, der dem Querschnitt des zu fertigenden distalen Endes des Außenschafts entspricht oder etwas größer als dieser ist. Das Frontbauteil wird insbesondere mittels Drahterodierens aus dem Werkstück erzeugt. Alternativ kann das Frontbauteil beispielsweise mittels eines spanabhebenden Verfahrens gefertigt werden.

Gerade bei einem geringen Querschnitt des zu fertigenden distalen Endes des Außenschafts und entsprechend kleinem Frontbauteil können die, verglichen mit anderen Fertigungsverfahren höheren Kosten des Erodierens oder des Laserschneidens eine untergeordnete Bedeutung haben. Eine Fertigung des Frontbauteils mittels Erodierens oder Laserschneidens kann jedoch eine sehr hohe Präzision ermöglichen, wie sie gerade für ein Frontbauteil für ein distales Ende eines Außenschafts mit geringem Querschnitt erforderlich ist.

Alternativ kann das Bereitstellen des Frontbauteils ein Bereitstellen eines Blechs und ein plastisches Verformen des Blechs umfassen.

Insbesondere wird das ursprünglich ebene Blech nur in einer Richtung bzw. in einer Ebene verformt, so dass die Flächennormalen des plastisch verformten Blechs parallel zu einer vorbestimmten Ebenen bzw. senkrecht zu einer vorbestimmten Geraden sind. Dabei weist das plastisch verformte Blech die Gestalt eines Ausschnitts eines Zylindermantels und eine verschwindende gaußsche Krümmung auf.

Das plastische Verformen eines Blechs kann eine besonders kostengünstige und gleichzeitig ausreichend präzise Fertigung des Frontbauteils ermöglichen.

Bei einem Verfahren, wie es hier beschrieben ist, kann das Bereitstellen des Frontbauteils ferner ein Erzeugen einer Durchgangsöffnung, die vorgesehen ist, um zumindest entweder eine Lichtaustrittsfläche in einem Beleuchtungsstrahlengang oder ein Fensterbauteil in einem Beobachtungsstrahlengang aufzunehmen, umfassen.

Eine oder mehrere Durchgangsöffnungen können vor oder nach den oben beschriebenen Schritten des Erodierens, Laserschneidens oder plastischen Verformens eines Blechs ausgeführt werden. Das Erzeugen von Durchgangsöffnungen vor dem Erodieren oder Laserschneiden kann den Vorteil aufweisen, dass das Werkstück zu diesem Zeitpunkt noch einfacher einzuspannen ist als das dünnwandige Frontbauteil. Vor dem plastischen Verformen eines Blechs können in dem noch ebenen Blech Durchgangsöffnungen beispielsweise durch Stanzen, Ätzen, Fräsen oder Erodieren kostengünstig und präzise erzeugt werden.

Bei einem Verfahren, wie es hier beschrieben ist, kann das Fügen des Frontbauteils an das Mantelbauteil ein Heften des Frontbauteils an das Mantelbauteil und ein anschließendes Fügen des Frontbauteils mit dem Mantelbauteil entlang der gesamten Fügefläche zwischen dem Mantelbauteil und dem Frontbauteil umfassen.

Das Heften des Frontbauteils an das Mantelbauteil erfolgt insbesondere an mehreren punktförmigen Stellen bzw. in mehreren kleinen Bereichen und insbesondere mittels Laserschweißens. Die Fügefläche weist insbesondere die Topologie eines geschlossenen Pfads bzw. eines Kreisrands auf. Das Fügen des Frontbauteils mit dem Mantelbauteil entlang der gesamten Fügefläche erfolgt insbesondere mittels Laserschweißens.

Ein Verfahren zum Fertigen eines Endoskops umfasst ein Bereitstellen eines Schaftrohres und ein Fertigen eines distalen Endes, wie es hier beschrieben ist, wobei das Mantelbauteil durch einen distalen Abschnitt des Schaftrohres gebildet oder separat gefertigt und anschließend an das Schaftrohr gefügt wird.

Ausführungsformen der vorliegenden Erfindung beruhen auf der Idee, ein distales Ende eines Außenschafts für ein Endoskop aus einem Rohr zu fertigen, dessen distaler Randbereich durch Bördeln zur Frontfläche oder einem Teil der Frontfläche des distalen Endes des Außenschafts umgeformt wird.

Ein Endoskop umfasst einen Außenschaft mit einem distalen Ende, dessen Oberfläche durch eine Mantelfläche und eine gekrümmte Frontfläche gebildet wird, wobei zumindest ein Teil der Mantelfläche aus einem Rohr gebildet ist, und wobei zumindest ein Teil der Frontfläche aus einem gebördelten Bereich des Rohrs gebildet ist.

Ein Außenschaft, dessen Frontfläche zumindest teilweise aus einem gebördelten Bereich eines Rohrs, das auch die Mantelfläche oder einen Teil der Mantelfläche bildet, gebildet ist, kann eine Dünnwandigkeit des distalen Endes des Außenschafts ermöglichen, die einen besonders großen Bauraum innerhalb des Außenschafts schafft. Gleichzeitig kann ein Bördeln des Rands eines Rohrs bei ausreichender Präzision kostengünstig sein.

Bei einem Endoskop kann ein Teil der Frontfläche des Außenschafts durch einen gebördelten Bereich eines Rohrs und ein anderer Teil der Frontfläche durch ein an das Rohr gefügtes Frontbauteil gebildet sein.

Eine Kombination eines oder mehrerer gebördelter Bereiche und eines oder mehrerer an das Rohr, insbesondere an die gebördelten Bereiche gefügten Frontbauteils kann hinsichtlich der erreichbaren Präzision und/oder hinsichtlich der Herstellungskosten vorteilhaft sein. Beispielsweise können schmale Abschnitte der Frontfläche am Rand von Öffnungen in der Frontfläche durch gebördelte Bereiche des Rohrs und andere, breitere oder großflächigere Bereiche der Frontfläche durch ein an das Rohr gefügtes Frontbauteil gebildet sein.

Bei einem Endoskop mit einem Außenschaft, dessen Frontfläche zumindest teilweise durch einen gebördelten Bereich eines Rohrs gebildet ist, kann die Frontfläche eine Öffnung in dem gebördelten Bereich aufweisen, die bereits vor dem Bördeln des Bereichs in dem zu bördelnden Bereich erzeugt wurde.

Durch das Erzeugen von Öffnungen bereits vor dem Bördeln kann die Verformbarkeit des zu bördelnden Bereichs erhöht werden. Ferner kann - abhängig von der Geometrie, dem Material und der verwendeten Verfahren - die Erzeugung einer Öffnung in einem zu bördelnden Bereich mit geringerem Aufwand möglich sein als die Erzeugung einer Öffnung in einem bereits gebördelden Bereich. Eine Nachbearbeitung der Öffnung nach dem Bördeln kann die erreichbare Präzision erhöhen.

Bei einem Endoskop mit einem Außenschaft, dessen Frontfläche zumindest teilweise durch einen gebördelten Bereich eines Rohrs gebildet ist, kann die Frontfläche eine Öffnung in dem gebördelten Bereich aufweisen, die nach dem Bördeln in dem gebördelten Bereich erzeugt wurde.

Eine Erzeugung einer Öffnung in der Frontfläche des Außenschafts nach dem Bördeln eines Bereichs des Rohrs kann insbesondere insoweit vorteilhaft sein, als am fertiggestellten Außenschaft ein gebördelter Bereich am Rand der Öffnung schmal und/oder die Öffnung klein ist.

Bei einem Verfahren zum Fertigen eines distalen Endes eines Außenschafts für ein Endoskop, bei dem die Oberfläche des distalen Endes des Außenschafts durch eine Mantelfläche und eine gekrümmte Frontfläche gebildet wird, werden zumindest ein Teil der Mantelfläche aus einem Rohr gebildet und zumindest ein Teil der Frontfläche durch Bördeln eines Bereichs des Rohrs gebildet.

Bei einem Verfahren zum Fertigen eines distalen Endes eines Außenschafts, bei dem zumindest ein Teil der Frontfläche durch Bördeln eines Bereichs des Rohrs gebildet wird, kann vor dem Bördeln eine Öffnung in dem zu bördelnden Bereich erzeugt werden.

Bei einem Verfahren zum Fertigen eines distalen Endes eines Außenschafts, bei dem zumindest ein Teil der Frontfläche durch Bördeln eines Bereichs des Rohrs gebildet wird, kann nach dem Bördeln eine Öffnung in dem zu bördelnden Bereich erzeugt werden.

Ein Rand eines zu bördelnden Bereichs des Rohrs kann - abhängig von der zu erzeugenden Geometrie und den mechanischen Eigenschaften des Rohrs und seines Materials - bereits vor dem Bördeln grob geformt, insbesondere beschnitten, werden, wobei eine Feinbearbeitung nach dem Bördeln erfolgt. Alternativ kann der Rand des zu bördelnden Bereichs bereits vor dem Bördeln so geformt, insbesondere beschnitten, werden, dass beim Bördeln ohne weiteren Bearbeitungsschritt die erwünschte Gestalt des Rand des gebördelten Bereichs entsteht. Entsprechendes gilt für Öffnungen in dem zu bördelnden Bereich bzw. in dem gebördelten Bereich des Rohrs.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Darstellung einer Ausführungsform des distalen Endes des Endoskops aus Figur 1
- Figur 3: eine weitere schematische Darstellung der Ausführungsform der Figur 2;
- Figur 4: eine weitere schematische Darstellung der Ausführungsform der Figuren 2 und 3;
- Figur 5: eine schematische Darstellung einer Kante der Ausführungsform der Figuren 2 bis 4;
- Figur 6: eine schematische Darstellung einer weiteren Ausführungsform des distalen Endes des Endoskops aus Figur 1;
- Figur 7: eine weitere schematische Darstellung der Ausführungsform der Figur 6;
- Figur 8: eine weitere schematische Darstellung der Ausführungsform der Figuren 6 und 7;
- Figur 9: eine schematische Darstellung einer Kante der Ausführungsformen der Figuren 6 bis 8;
- Figur 10: eine schematische Darstellung einer Ausführungsform des Frontbauteils der Figuren 2 bis 5;
- Figur 11: eine schematische Darstellung einer weiteren Ausführungsform des Frontbauteils der Figuren 2 bis 5;
- Figur 12: eine schematische Darstellung von Einrichtungen des Beleuchtungs- und des Beobachtungsstrahlengang;
- Figur 13: eine schematische Darstellung eines Mantelbauteils gemäß einer weiteren Ausführungsform;
- Figur 14: eine schematische Darstellung eines Frontbauteils gemäß der Ausführungsform der Figur 13;
- Figur 15: eine schematische Darstellung eines distalen Endes eines Außenschafts gemäß der Ausführungsform der Figuren 13 und 14;
- Figur 16: ein schematisches Flussdiagramm eines Verfahrens zum Fertigen eines distalen Endes für ein Endoskop;
- Figur 17: ein schematisches Flussdiagramm eines weiteren Verfahrens zum Fertigen eines distalen Endes für ein Endoskop;
- Figur 18: eine schematische Darstellung einer weiteren Ausführungsform des distalen Endes des Endoskops aus Figur 1.

### Beschreibung der Ausführungsformen

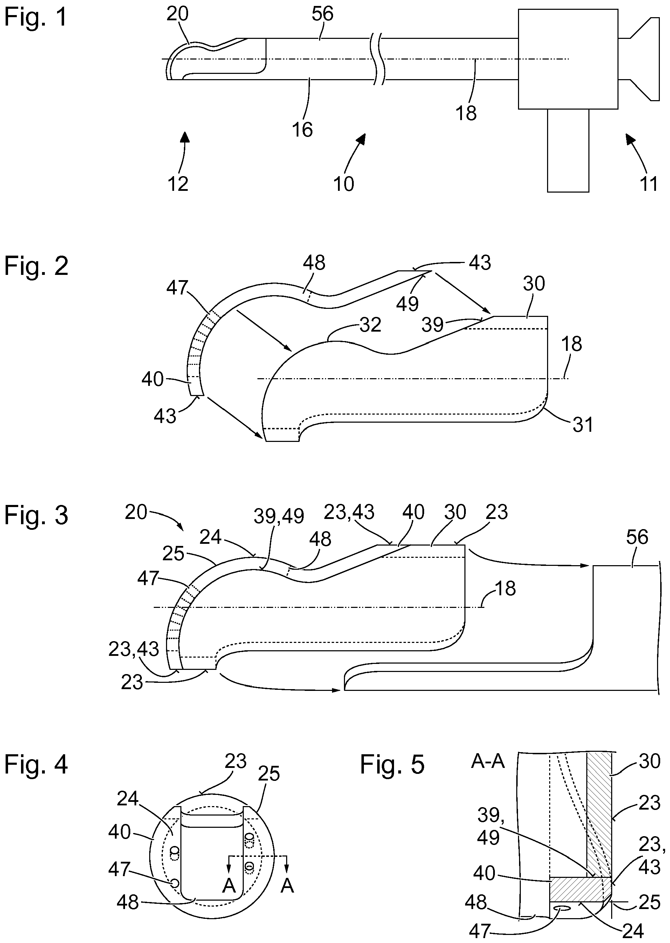

Figur 1 zeigt eine schematische Darstellung eines Endoskops 10 mit einem proximalen Ende 11 und einem distalen Ende 12. Am proximalen Ende 11 des Endoskops 10 sind ein Okular und eine Kupplung für ein Lichtleitkabel angedeutet, die im Folgenden nicht näher beschrieben werden. Ein Außenschaft 16 erstreckt sich vom proximalen Ende 11 bis zum distalen Ende 12 des Endoskops 10. Der Außenschaft 16 ist bei dem dargestellten Beispiel gerade und zylindrisch, insbesondere kreiszylindrisch ausgebildet. Im Fall einer zylindrischen Gestalt des Außenschafts 16 wird die Längsachse 18 des Endoskops insbesondere durch die Menge der Flächenmittelpunkte oder Flächenschwerpunkte der Querschnittsflächen des Außenschafts 16 gebildet. Soweit der Außenschaft 16 des Endoskops 10 abweichend von der Darstellung in Figur 1 gekrümmt ist, ist mit der Längsachse 18 die Längsachse am distalen Ende 12 des Endoskops 10 gemeint.

Bei dem in Figur 1 dargestellten Endoskop weist der Außenschaft 16 ein distales Ende 20 auf, das als separates Bauteil gefertigt und danach an das den Außenschaft 16 überwiegend bildende Schaftrohr 56 gefügt ist. Ausführungsbeispiele des distalen Endes 20 des Außenschafts 16 und seiner Fertigung sind nachfolgend anhand der weiteren Figuren beschrieben.

Die Figuren 2 bis 5 zeigen schematische Darstellungen einer Ausführungsform des distalen Endes 20 des Endoskops 10 aus Figur 1. Die Figuren 2 und 3 zeigen Darstellungen, bei denen die Zeichenebenen parallel zur Zeichenebene der Figur 1 und zur Längsachse 18 des Endoskops 10 sind. Die Zeichenebene der Figur 4 ist senkrecht zu den Zeichenebenen der Figuren 1 bis 3 und zur Längsachse 18 des Endoskops 10. Figur 5 zeigt eine schematische Darstellung eines Schnitts entlang einer Ebene A-A parallel zur Längsachse 18 des Endoskops 10 und senkrecht zu den Zeichenebenen der Figuren 1 bis 4. Die Schnittebene A-A ist in Figur 4 angedeutet. In den Figuren 2 bis 5 sind in gestrichelten Linien Konturen angedeutet, die in den Darstellungen der Figuren 2 bis 4 eigentlich nicht sichtbar sind bzw. bei der Darstellung in Figur 5 zwischen dem Betrachter und der Schnittebene A-A liegen.

Figur 2 zeigt eine schematische Darstellung eines Mantelbauteils 30 und eines Frontbauteils 40 die dafür vorgesehen sind - wie durch Pfeile in Figur 2 angedeutet - zusammengesetzt und gefügt zu werden. Das Mantelbauteil 30 ist aus einem Rohr mit kreisförmigem Querschnitt geschnitten und weist entsprechend die Gestalt eines Ausschnitts einer Kreiszylinder-Mantelfläche auf. Sowohl der proximale Rand 31 als auch der distale Rand 32 des Mantelbauteils 30 liegen jeweils nicht in einer Ebene. Die Gestalt des proximalen Rands 31 des Mantelbauteils 30 ist an die Gestalt des distalen Rands des in den Figuren 1 und 3 gezeigten Schaftrohres 56 angepasst. Die Gestalt des distalen Rands 32 des Mantelbauteils 30 ist an die Gestalt des Frontbauteils 40 angepasst bzw. entspricht dieser.

Das Frontbauteil 40 weist die Gestalt eines Ausschnitts eines Zylindermantels mit nicht-kreisförmigem Querschnitt und Symmetrieachse senkrecht zur Längsachse 18 und zur Zeichenebene der Figur 2 auf, so dass alle Flächennormalen dieses Ausschnitts der Zylindermantelfläche parallel zur Zeichenebene der Figur 2 sind.

Das Frontbauteil 40 weist am Rand eine umlaufende streifenförmige Randfläche 43 auf, die vorgesehen und ausgebildet ist, am fertigen distalen Ende einen Teil von dessen Mantelfläche zu bilden. Ferner weist das Frontbauteil 40 mehrere Öffnungen 47 auf, die vorgesehen und ausgebildet sind, um am fertigen Endoskop jeweils in einem Zweig eines Beleuchtungsstrahlengangs zu liegen bzw. eine Lichtaustrittsfläche aufzunehmen. Ferner weist das Frontbauteil 40 eine Öffnung 48 auf, die vorgesehen und ausgebildet ist, um am fertigen Endoskop im Beobachtungsstrahlengang zu liegen, insbesondere ein Fensterbauteil mit der Gestalt eines Ausschnitts eines Kreiszylindermantels aufzunehmen. Die Ausdehnungen und Formen der Öffnungen 47, 48 sowie ihre Anordnung ist insbesondere in Figur 4 erkennbar.

Wie durch Pfeile in Figur 2 angedeutet, sind das Mantelbauteil 30 und das Frontbauteil 40 vorgesehen, um aneinandergesetzt und gefügt zu werden, um das in Figur 3 erkennbare distale Ende 20 zu bilden. Zum Fügen sind insbesondere eine Fügefläche 39 am Mantelbauteil 30 und eine korrespondierende Fügefläche 49 am Frontbauteil 40 vorgesehen und ausgebildet. Die Fügefläche 39 am Mantelbauteil 30 und die Fügefläche 49 am Frontbauteil 40 weisen bei diesem Ausführungsbeispiel jeweils die Gestalt eines Streifens mit variierender Breite entlang dem distalen Rand 32 des Mantelbauteils 30 bzw. entlang dem Rand des Frontbauteils 40 auf. Die Fügefläche 39 des Mantelbauteils 30 und die Fügefläche 49 des Frontbauteils 40 sind jeweils parallel zur Normalen der Zeichenebene der Figur 2. Anders ausgedrückt sind alle Flächennormalen der Fügefläche 39 des Mantelbauteils 30 und der Fügefläche 49 des Frontbauteils 40 parallel zur Zeichenebene der Figur 2.

Figur 3 zeigt eine schematische Darstellung des distalen Endes 20, das aus den oben anhand der Figur 2 beschriebenen Bestandteilen Mantelbauteil 30 und Frontbauteil 40 gebildet ist. Das distale Ende 20 weist eine Mantelfläche 23, eine Frontfläche 24 und eine Kante 25 zwischen der Mantelfläche 23 und der Frontfläche 24 auf. Die Mantelfläche 23 weist die Gestalt eines Ausschnitts bzw. eines Teilbereichs eines Kreiszylindermantels auf, alle Flächennormalen der Mantelfläche 23 sind also senkrecht zur Längsachse 18. Die Flächennormalen der Frontfläche 24 sind parallel zur Zeichenebene der Figur 3 bzw. senkrecht zur Flächennormalen der Zeichenebene der Figur 3. Die Kante 25 zwischen der Mantelfläche 23 und der Frontfläche 24 weist eine ringförmige Topologie auf. Die Mantelfläche 23 wird überwiegend vom Mantelbauteil 30 bzw. von einem Teil von dessen Oberfläche gebildet. Ein schmaler, an die Kante 25 angrenzender Randbereich der Mantelfläche 23 wird durch eine Randfläche 43 des Frontbauteils 40 gebildet. Der Abstand der Kante 25 von der Fügefläche 39, 49 zwischen dem Mantelbauteil 30 und dem Frontbauteil 40 (gemessen in Richtung parallel zur lokalen Flächennormalen der Fügefläche 39, 49) entspricht der Wandstärke des Frontbauteils 40. Die Breite b der streifenförmigen Randfläche 43 variiert abhängig vom Winkel α zwischen der Mantelfläche 23 und der Frontfläche 24 und ist mindestens so groß wie die Wandstärke d des Frontbauteils 40, b = d/sin(α).

In Figur 3 ist ferner das distale Ende des Schaftrohres 56 dargestellt. Durch Pfeile ist angedeutet, wie das distale Ende 20 an das Schaftrohr 56 angesetzt werden kann, um den Außenschaft 16 (vgl. Figur 1) für ein Endoskop zu bilden.

In der schematischen Darstellung in Figur 4 mit Blickrichtung parallel zur Längsachse 18 (vgl. Figuren 1 bis 3) auf das distale Ende 20 ist der kreisförmige Querschnitt des distalen Endes 20 erkennbar. Unmittelbar sichtbar sind ausschließlich das Frontbauteil 40 und die durch das Frontbauteil 40 gebildete Frontfläche 24 mit Öffnungen 47 für Teilstränge des Beleuchtungsstrahlengangs und einer Öffnung 48 für ein Fensterbauteil im Beobachtungsstrahlengang. In der dargestellten Projektion erscheinen sowohl die Mantelfläche 23 als auch die Kante 25 zwischen Mantelfläche 23 und Frontfläche 24 als Kreise. In gestrichelten Linien sind Konturen des in Figur 4 nicht sichtbaren Mantelbauteils 30 angedeutet.

Figur 5 zeigt eine schematische vergrößerte Darstellung eines Schnitts entlang der in Figur 4 angedeuteten Ebene A-A. In Figur 5 sind insbesondere das Mantelbauteil 30, das Frontbauteil 40 und die Fügefläche 39, 49 zwischen dem Mantelbauteil 30 und dem Frontbauteil 40 jeweils im Querschnitt deutlich erkennbar. Ferner sind eine Öffnung 47 im Beleuchtungsstrahlengang und die angeschnittene Öffnung 48 im Beobachtungsstrahlengang erkennbar.

In der vergrößerten Darstellung der Figur 5 ist ferner erkennbar, dass die Kante zwischen der Mantelfläche 23 und der Frontfläche 24 gebrochen ist bzw. eine Fase aufweist. Alternativ kann die Kante abgerundet sein. Wie bereits erwähnt, wird in diesem Fall als Kante 25, von der beispielsweise ein Abstand der Fügefläche 39, 49 bestimmt wird, die Schnittlinie zwischen der fortgesetzten Mantelfläche 23 und der fortgesetzten Frontfläche 24 angesehen. Die Fortsetzungen der Mantelfläche 23 und der Frontfläche 24 sind in Figur 5 durch kurze gerade Linien angedeutet, die sich im Schnittpunkt der Kante 25 mit der Ebene A-A schneiden.

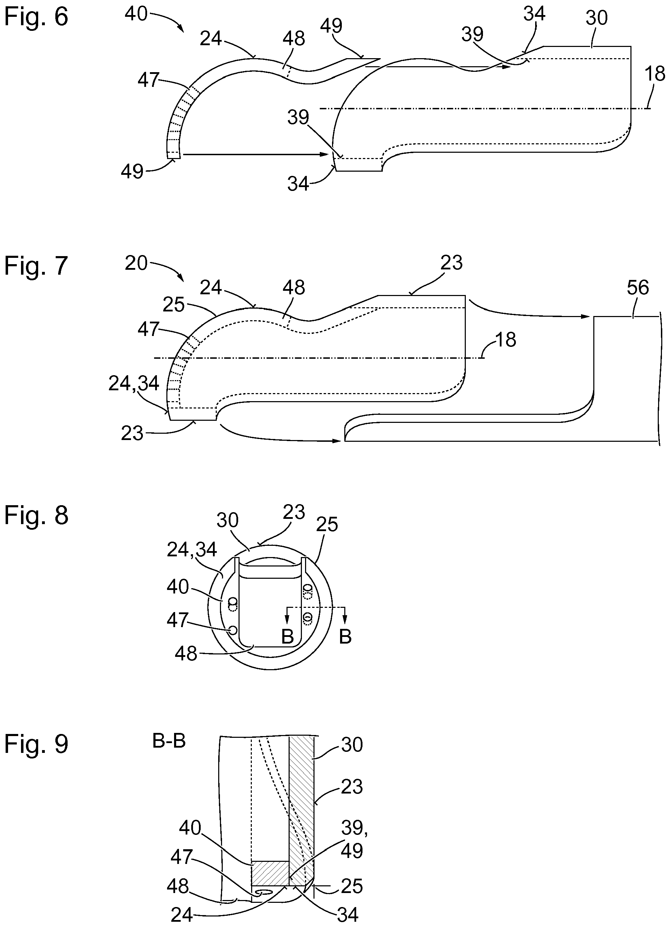

Die Figuren 6 bis 8 zeigen schematische Darstellungen eines weiteren Ausführungsbeispiels des distalen Endes 20 des Außenschafts 16 des oben anhand der Figur 1 dargestellten Endoskops 10. Hinsichtlich der Art der Darstellung und der Zeichenebene bzw. der Schnittebene entsprechen einander jeweils die Figuren 2 und 6; 3 und 7; 4 und 8; 5 und 9.

Das Ausführungsbeispiel der Figuren 6 bis 9 ähnelt in einigen Merkmalen dem Ausführungsbeispiel der Figuren 2 bis 5. Insbesondere in den Figuren 6 und 9 sind im Vergleich zu den Figuren 2 und 5 Unterschiede erkennbar. Beim Ausführungsbeispiel der Figuren 6 bis 9 reicht das Mantelbauteil 30 bis zur Frontfläche 24 des distalen Endes 20. Die gesamte Mantelfläche 23 des distalen Endes 20 ist somit durch einen Bereich der Oberfläche des Mantelbauteils 30 gebildet. Eine streifenförmige Stirnfläche 34 des Mantelbauteils 30 bildet ferner einen schmalen, umlaufenden und an die Kante 25 angrenzenden Randstreifen der Frontfläche 24 des distalen Endes 20. Entsprechend weisen die Fügefläche 39 des Mantelbauteils 30 und die Fügefläche 49 des Frontbauteils 40 jeweils die Gestalt eines streifenförmigen Ausschnitts mit ringförmiger Topologie eines Kreiszylindermantels auf. Anders ausgedrückt sind die Fügeflächen 39, 49 des Mantelbauteils 30 und des Frontbauteils 40 bei dem Ausführungsbeispiel der Figuren 6 bis 9 parallel zur Mantelfläche 23 und zur Längsachse 18. Eine Konsequenz ist, dass in der Darstellung in Figur 8 außer dem Frontbauteil 40 auch die Stirnfläche 34 des Mantelbauteils 30 unmittelbar sichtbar ist.

Bei den Ausführungsbeispielen der Figuren 2 bis 9 ist vorgesehen, dass das distale Ende 20 als separates Bauteil gefertigt und danach an das in den Figuren 1, 3 und 7 dargestellte Schaftrohr 56 angesetzt und mit diesem gefügt wird. Dies ermöglicht insbesondere ein Einsetzen von in den Figuren 1 bis 9 nicht dargestellten Einrichtungen des Beobachtungsstrahlengangs, Einrichtungen des Beleuchtungsstrahlengangs und anderen Einrichtungen vor dem Fügen des distalen Endes 20 an das Schaftrohr 56.

Alternativ werden das Mantelbauteil 30 und das Schaftrohr 56 von vornherein einstückig gefertigt. Dadurch kann die Anzahl der Fügeflächen und die Anzahl der Fügevorgänge reduziert werden.

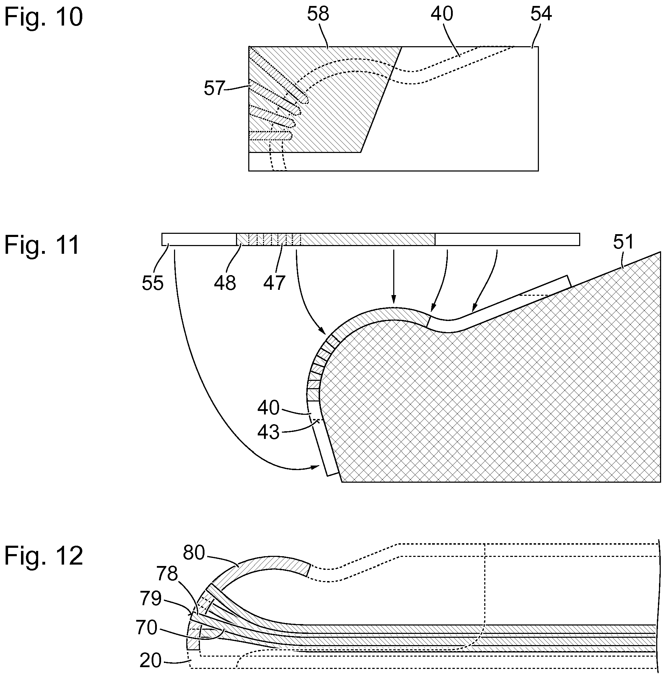

Figur 10 zeigt eine schematische Darstellung eines Werkstücks 54, aus dem das Frontbauteil 40 des Ausführungsbeispiels aus den Figuren 2 bis 5 erzeugt werden kann. Das Werkstück 54 weist ursprünglich insbesondere die Gestalt eines Vollzylinders mit dem Querschnitt des zu erzeugenden distalen Endes 20 oder einem etwas größeren Querschnitt auf.

Zunächst werden im Werkstück 54 Ausnehmungen 57 (insbesondere kreiszylindrische Bohrungen) für die Öffnungen 47 für den Beleuchtungsstrahlengang und eine Ausnehmung 58 für die Öffnung 48 im Beobachtungsstrahlengang (vgl. Figuren 2 bis 5) erzeugt. Die Ausnehmungen 57, 58 werden insbesondere mittels eines spanabhebenden Verfahrens oder durch ein Erodierverfahren erzeugt. Danach wird die in Figur 10 in gestrichelter Linie dargestellte Kontur des Frontbauteils 40 durch Drahterodieren erzeugt, wobei der Draht senkrecht zur Zeichenebene der Figur 10 verläuft. Alternativ wird das Frontbauteil 40 durch Laserschneiden oder mittels eines spanabhebenden Verfahrens aus dem Werkstück 54 erzeugt. Die Öffnungen 57, 58 können alternativ teilweise oder vollständig nach dem Erzeugen der in Figur 10 durch gestrichelte Linien angedeuteten Kontur des Frontbauteils 40 erzeugt werden.

Figur 11 zeigt eine schematische Darstellung einer Matrize 51, eines Blechs 55 und des mittels der Matrize 51 durch plastisches Verformen des Blechs 55 erzeugten Frontbauteils 40 für das Ausführungsbeispiel der Figuren 2 bis 5. Die Zeichenebene der Figur 11 ist parallel zu den Zeichenebenen der Figuren 1 bis 3. Durch Pfeile in Figur 11 ist angedeutet, wie das Blech 55 plastisch verformt wird. Die Öffnungen 47, 48 für den Beleuchtungsstrahlengang und für Teilstränge des Beobachtungsstrahlengang können, wie in Figur 11 angedeutet, vor dem plastischen Verformen des Blechs 55 in diesem erzeugt werden, beispielsweise durch Stanzen, Ätzen, Erodieren, Laserschneiden oder mittels eines spanabhebenden Verfahrens. Alternativ werden die Öffnungen 47, 48 abweichend von der Darstellung in Figur 11 erst nach dem plastischen Verformen des Blechs 55 erzeugt.

Die in Figur 11 durch gestrichelte Linien angedeutete Randfläche 43 wird nach dem plastischen Verformen des Blechs 55 vor dem Fügen oder nach dem Fügen des Frontbauteils 40 an das Mantelbauteil 30 (vgl. Figur 2) erzeugt, beispielsweise durch Erodieren, Laserschneiden oder Schleifen.

Figur 12 zeigt eine schematische Darstellung von Lichtleitern 70 und eines Fensterbauteils 80, die in das distale Ende 20 gemäß einem der Ausführungsbeispiele der Figuren 2 bis 11 eingesetzt werden können. Die Zeichenebene der Figur 12 entspricht den Zeichenebenen der Figuren 1 bis 3, 6, 7, 10 und 11 oder ist parallel zu diesen. Das distale Ende 20 und das sich proximal anschließende Schaftrohr des Außenschafts sind lediglich in gestrichelten Linien angedeutet. Bezogen auf die Blickrichtung, die der Darstellung in Figur 12 zugrunde liegt, sind die distalen Enden 78 zweier Lichtleiter 70 vor und die distalen Enden zweier weiterer Lichtleiter 70 hinter dem Fensterbauteil angeordnet.

Die Lichtleiter 70 bilden bzw. definieren Teilstränge eines Beleuchtungsstrahlengangs zum Leiten von Beleuchtungslicht. Insbesondere umfasst jeder Lichtleiter 70 ein Bündel von biegeelastischen Glasfasern oder anderen Lichtwellenleitern. Distale Enden 78 der Lichtleiter 70 sind in je eine der insbesondere in den Figuren 2 bis 9 erkennbaren Öffnungen 47 eingesetzt. Lichtaustrittsflächen 79 der Lichtleiter 70 sind insbesondere bündig oder im Wesentlichen bündig mit der Frontfläche 24 (vgl. insbesondere die Figuren 3 und 7) ausgebildet. Die Lichtaustrittsflächen 79 können nach dem Einsetzen und Verkleben oder anderen Fügen der distalen Enden 78 der Lichtleiter 70 in je eine Öffnung 47 des Frontbauteils 40 durch schleifen und polieren erzeugt werden.

Das Fensterbauteil 80 bildet einen Bestandteil eines Beobachtungsstrahlengangs zum Leiten bzw. Übertragen von Licht, das von einem mittels des Endoskops 10 (vgl. Figur 1) zu beobachtenden Objekt ausgeht. Das Fensterbauteil 80 weist ähnlich wie das Frontbauteil 40 (vgl. insbesondere die Figuren 2, 3, 6) die Gestalt eines Ausschnitts eines Zylindermantels auf. Insbesondere weist das Fensterbauteil 80 die Gestalt eines Kreiszylindermantels auf. Alternativ weist das Fensterbauteil die Gestalt eines Ausschnitts aus einer Oberfläche eines Ellipsoids, insbesondere aus einer Kugeloberfläche, auf. Mit der Gestalt eines Ausschnitts eines Kreiszylindermantels oder eines Ellipsoids ist das Fensterbauteil 80 insbesondere für ein Endoskop mit einstellbarer Blickrichtung geeignet.

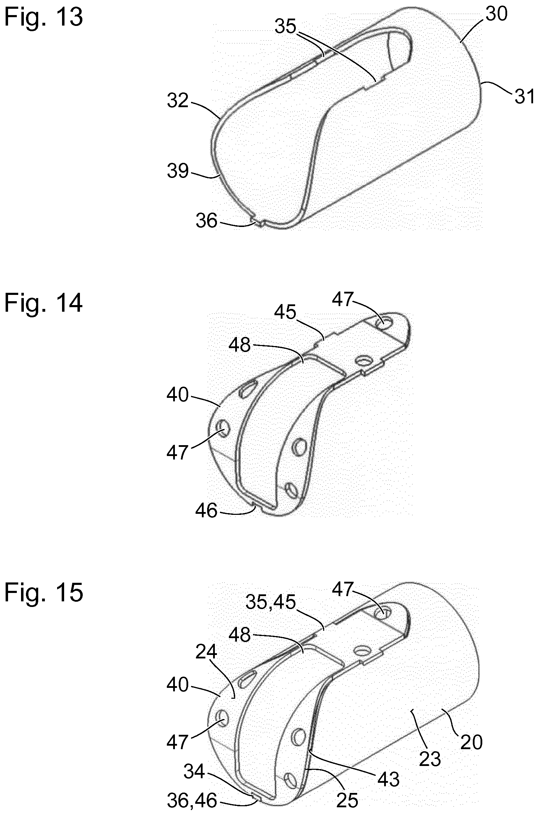

Die Figuren 13 bis 15 zeigen schematische axonometrische Darstellungen einer weiteren Ausführungsform eines distalen Endes 20 für einen Außenschaft 16 eines Endoskops 10, wie es oben anhand der Figur 1 dargestellt ist. Das distale Ende 20 umfasst ein Mantelbauteil 30, das in Figur 13 dargestellt ist, und ein Frontbauteil 40, das in Figur 14 dargestellt ist. Figur 15 zeigt das Frontbauteil 40 an das Mantelbauteil 30 angesetzt. Das Frontbauteil 40 ist insbesondere - ähnlich wie oben anhand der Figur 11 dargestellt - durch plastisches Verformen eines ursprünglich ebenen Blechs gefertigt, wobei die Öffnungen 47, 48 bereits vor dem plastischen Verformen durch stanzen, ätzen, laserschneiden oder auf andere Weise in das noch ebene Blech eingebracht worden sein können.

Das Ausführungsbeispiel der Figuren 13 bis 15 zeigt, dass das die Kante 25 zwischen Mantelfläche 23 und Frontfläche 24 nicht nur entweder ausschließlich durch das Mantelbauteil 30 oder ausschließlich durch das Frontbauteil 40 gebildet sein kann. Bei dem Ausführungsbeispiel der Figuren 13 bis 15 ist die Kante 25 insbesondere distal überwiegend durch das Frontbauteil 40 gebildet. Das Frontbauteil 40 weist jedoch eine Ausnehmung 46 auf, in das eine Zinke bzw. ein Vorsprung 36 am distalen Rand 32 des Mantelbauteils 30 eingreift. Im Bereich der Zinke bzw. des Vorsprungs 36 bildet das Mantelbauteil 30 die Kante 25.

Ferner weist in einem proximalen Bereich das Mantelbauteil 30 Ausnehmungen 35 auf, in die je eine Zinke bzw. ein Vorsprung 45 am Frontbauteil 40 eingreift. In dem proximalen Bereich in der Umgebung der Ausnehmung 35 wird die Kante 25 überwiegend durch das Mantelbauteil 30 oder durch ein Lot oder eine Schweißnaht, das bzw. die in dem in Figur 15 dargestellten Zustand noch nicht vorhanden ist, gebildet. Jeweils im Bereich einer Ausnehmung 35 und eines Vorsprungs 45 selbst wird die Kante durch den Vorsprung 45 gebildet. Dabei sind die Vorsprünge 45 in Figur 15 noch über die Mantelfläche 24 leicht überstehend dargestellt. Diese Überstände werden nach dem Fügen des Frontbauteils 40 an das Mantelbauteil 30 beispielsweise durch abschleifen oder abdrehen entfernt.

Die Ausnehmungen 35, 46 und die in diese eingreifenden korrespondierenden Vorsprünge 36, 45 schaffen einen Formschluss zwischen Mantelbauteil 30 und Frontbauteil 40. Dieser Formschluss kann die relative Anordnung und Ausrichtung von Mantelbauteil 30 und Frontbauteil 40 präzise definieren. Dadurch können die Fertigung des distalen Endes 20 vereinfacht und die dabei erreichbare Präzision erhöht werden.

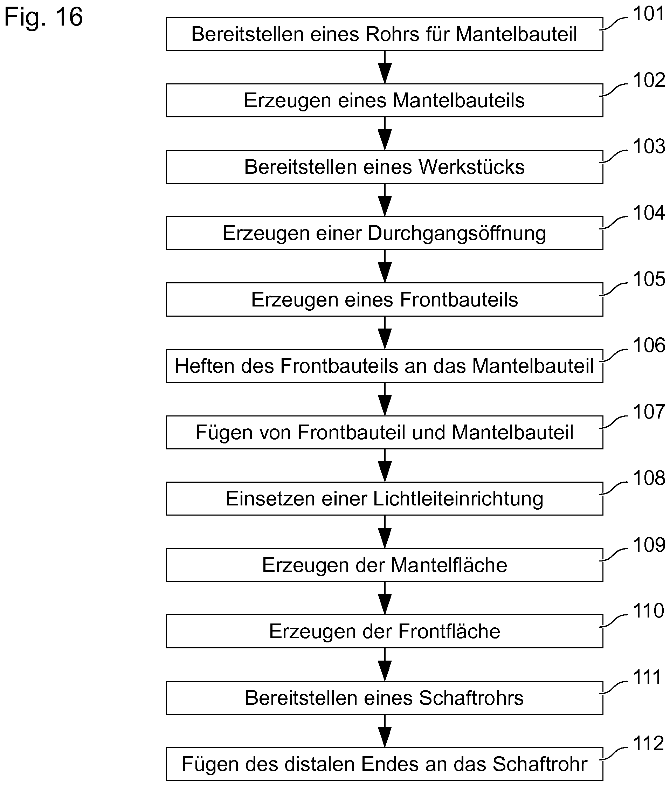

Figur 16 zeigt ein schematisches Flussdiagramm eines Verfahrens zum Fertigen eines Endoskops. Das in Figur 16 dargestellte Verfahren ist auch zum Fertigen von distalen Enden von Außenschäften geeignet, die von den oben anhand der Figuren 1 bis 15 dargestellten Ausführungsbeispielen abweichende Merkmale und Eigenschaften aufweisen. Trotzdem werden nachfolgend Bezugszeichen der Figuren 1 bis 15 verwendet, um das Verständnis zu vereinfachen.

Bei einem ersten Schritt 101 wird ein Rohr bereitgestellt, aus dem ein Mantelbauteil zu erzeugen ist. Bei einem zweiten Schritt 102 wird aus dem beim ersten Schritt 101 bereitgestellten Rohr ein Mantelbauteil 30 erzeugt, insbesondere durch Erzeugen eines proximalen Rands 31 und eines distalen Rands 32.

Bei einem dritten Schritt 103 wird ein Werkstück 54 bereitgestellt, aus dem ein Frontbauteil zu erzeugen ist. Bei einem vierten Schritt 104 werden Ausnehmungen 57, 58 erzeugt, die am fertigen Frontbauteil 40 Durchgangsöffnungen 47, 48 bilden. Bei einem fünften Schritt 105 wird das Frontbauteil 40 aus dem Werkstück 54 erzeugt, insbesondere durch Drahterodieren, Laserschneiden oder mittels eines spanabhebenden Verfahrens. Abweichend von der Darstellung in Figur 16 können Durchgangsöffnungen 57, 58 im Frontbauteil 40 nach dem fünften Schritt 105 erzeugt werden.

Bei einem sechsten Schritt 106 wird das beim dritten Schritt 103, beim vierten Schritt 104 und beim fünften Schritt 105 erzeugte Frontbauteil 40 an das beim ersten Schritt 101 und beim zweiten Schritt 102 erzeugte Mantelbauteil 30 geheftet, insbesondere durch Laserschweißen an mehreren Punkten bzw. in mehreren kleinen Bereichen. Bei einem siebten Schritt 107 werden das Frontbauteil 40 und das Mantelbauteil 30 gefügt. Dabei werden insbesondere eine umlaufende Fügefläche 39 am Mantelbauteil 30 und eine korrespondierende umlaufende Fügefläche 49 am Frontbauteil 40 gefügt. Die Fügefläche 39, 49 zwischen Mantelbauteil 30 und Frontbauteil 40 weist einen Abstand von einer Kante 25 zwischen einer Mantelfläche 23 und einer Frontfläche 24 des Endes 20 des Außenschafts 16 auf, der nicht größer als die Wandstärke des Mantelbauteils 30 oder nicht größer als die Wandstärke des Frontbauteils 40 ist.

Bei einem achten Schritt 108 wird eine Lichtleiteinrichtung aus einem oder mehreren Lichtleitern 70 in das distale Ende 20 eingesetzt. Dabei werden insbesondere Bündel von Lichtleitfasern in Durchgangsöffnungen 47 im Frontbauteil 40 eingesetzt und dort beispielsweise verklebt oder verkittet. Bei einem neunten Schritt 109 wird die Mantelfläche 23 des distalen Endes 20 erzeugt oder überarbeitet, insbesondere durch Abdrehen oder Schleifen. Der neunte Schritt 109 kann abweichend von der Darstellung in Figur 16 vor dem achten Schritt 108 ausgeführt werden. Bei einem zehnten Schritt 110 wird eine Frontfläche 24 des distalen Endes 20 erzeugt, insbesondere durch Schleifen. Dabei können gleichzeitig Lichtaustrittsflächen 79 von beim achten Schritt 108 eingesetzten Bündeln von Lichtleitfasern erzeugt werden, insbesondere durch Polieren.

Bei einem elften Schritt 111 wird ein Schaftrohr 56 bereitgestellt. Bei einem zwölften Schritt 112 wird das distale Ende 20 an das Schaftrohr 56 gefügt, um einen Außenschaft 16 für ein Endoskop 10 zu bilden. Alternativ und abweichend von der Darstellung in Figur 16 kann das distale Ende 20 bzw. dessen Mantelbauteil 30 von vornherein einstückig mit dem Schaftrohr 56 ausgeführt sein. In diesem Fall entfällt insbesondere der zwölfte Schritt 112.

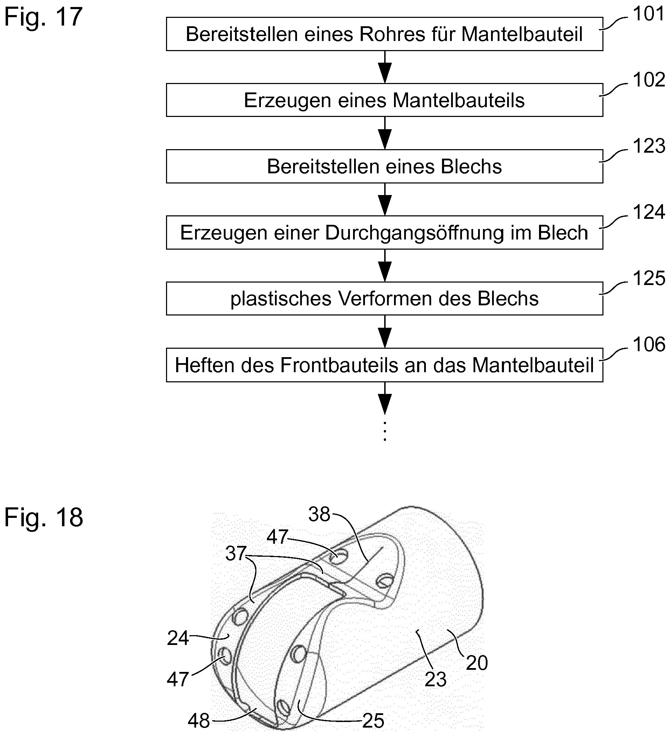

Figur 17 zeigt ein schematisches Flussdiagramm eines weiteren Verfahrens zum Fertigen eines Endoskops. Der erste Schritt 101, der zweite Schritt 102 und die Schritte ab dem sechsten Schritt 106 entsprechen den gleichnamigen oben anhand der Figur 16 dargestellten Schritten. Das in Figur 17 dargestellte Verfahren unterscheidet sich von dem in Figur 16 dargestellten Verfahren im dritten Schritt 123, im vierten Schritt 124 und im fünften Schritt 125.

Beim dritten Schritt 123 wird ein insbesondere zunächst ebenes Blech 55 bereitgestellt. Beim vierten Schritt 124 werden eine oder mehrere Durchgangsöffnungen 47, 48 in dem Blech 55 erzeugt. Beim fünften Schritt 125 wird das Blech 55 plastisch verformt, um das Frontbauteil 40 zu bilden, insbesondere mittels einer Matrize 51. Alternativ und abweichend von der Darstellung in Figur 17 kann die Reihenfolge des vierten Schritts 124 und des fünften Schritts 125 vertauscht werden.

Bei beiden anhand der Figuren 16 und 17 dargestellten Verfahren sind einige Verfahrensschritte optional, beispielsweise der sechste Schritt 106.

Figur 18 zeigt eine schematische axonometrische Darstellung einer weiteren Ausführungsform eines distalen Endes 20 für einen Außenschaft 16 eines Endoskops 10, wie es oben anhand der Figur 1 dargestellt ist. Die Ausführungsform der Figur 18 ähnelt in einigen Merkmalen den Ausführungsformen der Figuren 2 bis 9 und 13 bis 15, insbesondere hinsichtlich der Anordnung von Öffnungen 47 für einen Beleuchtungsstrahlengang und der Anordnung und Gestalt einer Öffnung 48 für einen Beobachtungsstrahlengang.

Das in Figur 18 dargestellte distale Ende 20 für einen Außenschaft 16 eines Endoskops 20 unterscheidet sich von den Ausführungsformen der Figuren 2 bis 9 und 13 bis 15 insbesondere dadurch, dass sowohl die Mantelfläche 23 als auch die Frontfläche 24 aus einem Rohr bzw. einem einzigen Rohrstück gebildet sind. Die Mantelfläche 23 wird durch das unveränderte Rohr gebildet. Die Frontfläche 24 wird durch gebördelte Bereiche 37 des Rohrs gebildet.

Nahe dem in Figur 18 unten dargestellten distalen Rand der Öffnung 48 für ein Fensterbauteil im Beobachtungsstrahlengang ist der gebördelte Bereich 37 sehr schmal. Auf ein Bördeln kann in diesem Bereich unter Umständen ganz verzichtet werden, insbesondere wenn ein geeignetes Fügeverfahren zum Einsetzen des Fensterbauteils in die Öffnung 48 verwendet wird und die Öffnung 48 und das einzusetzende Fensterbauteil eine geeignete Gestalt aufweisen.

Seitlich der Öffnung 48 sind gebördelte Bereiche 37 variierender Breite vorgesehen, die ferner jeweils eine oder mehrere für einen Beleuchtungsstrahlengang vorgesehene Öffnungen 47 aufweisen können. Zwischen diesen gebördelten Bereichen 37 seitlich der Öffnung 48 und der Mantelfläche 23 ist ferner eine abgerundete Kante 25 erkennbar. Eine Abrundung der Kante 25 kann den Vorgang des Bördeln vereinfachen und hinsichtlich der nach dem Bördeln vorliegenden Festigkeit des Materials des Rohrs vorteilhaft sein.

Proximal und seitlich der Öffnung 48 liegt ein verhältnismäßig großflächiger gebördelter Bereich 37 vor. Um das erforderliche Maß der Stauchung beim Bördeln dieses Bereichs 37 zu verringern, kann eine Nahtstelle 38 vorgesehen sein, an der zwei vor dem Bördeln näherungsweise V-förmige Ränder des Rohrs nach dem Bördeln aneinander stoßen und gefügt werden.

Bei der Fertigung des in Figur 18 gezeigten distalen Endes 20 können die Öffnungen 47 oder ein Teil der Öffnungen und der Rand der Öffnung 48 bereits vor dem Bördeln in den zu bördelnden Bereichen des noch ungebördelten Rohrs erzeugt werden. Bei der Erzeugung der Öffnungen 47 und des Rands ist zu berücksichtigen, dass diese beim Bördeln verformt werden, insbesondere Scherung, Stauchung und Dehnung ausgesetzt sein können. Um die geometrische Präzision zu erhöhen, können sowohl die Öffnungen 47 als auch der Rand der Öffnung 48 nach dem Bördeln nachbearbeitet werden. Alternativ können die Öffnungen 47 oder ein Teil der Öffnungen 47 und/oder der Rand 48 erst nach dem Bördeln erzeugt werden.

Alternativ kann abweichend von der Darstellung in Figur 18 ein Teil der Frontfläche durch ein zunächst separat hergestelltes und dann an das Rohr gefügtes Frontbauteil gebildet werden. Beispielsweise werden die seitlich der Öffnung 48 liegenden Bereiche der Frontfläche 24 durch Bördeln erzeugt, und der größere Bereich proximal der Öffnung 48 zumindest teilweise durch ein Frontbauteil gebildet. Dieses Frontbauteil kann bis zur Kante 25 zwischen Mantelfläche 23 und Frontfläche 24 reichen und unmittelbar mit ungebördelten Bereichen des Rohrs gefügt werden. Ferner kann das Frontbauteil alternativ oder an anderen Stellen mit gebördelten Bereichen des Rohrs gefügt werden.

Zum Erzeugen oder Nachbearbeiten der Öffnungen 47 oder des Rands 48 können, abhängig davon, ob vor oder nach dem Bördeln, spanabhebende Verfahren wie Bohren, Fräsen oder Schleifen, oder andere Verfahren wie Laserschneiden, Stanzen, Drahterodieren verwendet werden.

### Bezugszeichen

- 10: Endoskop
- 11: proximales Ende des Endoskops 10
- 12: distales Ende des Endoskops 10
- 16: Außenschaft des Endoskops 10
- 18: Längsachse des Endoskops 10
- 20: distales Ende des Außenschafts 16
- 23: Mantelfläche des distalen Endes 20
- 24: Frontfläche des distalen Endes 20
- 25: Kante zwischen Mantelfläche 23 und Frontfläche 24
- 30: Mantelbauteil des distalen Endes 20
- 31: proximaler Rand des Mantelbauteils 30
- 32: distaler Rand des Mantelbauteils 30
- 34: Stirnfläche des Mantelbauteils 30
- 35: Ausnehmung am Rand des Mantelbauteils 30
- 36: Vorsprung am Rand des Mantelbauteils 30
- 37: gebördelter Bereich
- 38: Nahtstelle zwischen Abschnitten des gebördelten Bereichs 37
- 39: Fügefläche am Mantelbauteil 30
- 40: Frontbauteil des distalen Endes 20
- 43: Randfläche des Frontbauteils 40
- 45: Vorsprung am Rand des Frontbauteils 40
- 46: Ausnehmung am Rand des Frontbauteils 40
- 47: Öffnung für Beleuchtungsstrahlengang
- 48: Öffnung für Beobachtungsstrahlengang
- 49: Fügefläche am Frontbauteil 40
- 51: Matrize
- 54: Werkstück für Frontbauteil 40
- 55: Blech
- 56: Schaftrohr
- 57: Ausnehmung für Öffnung 47 für Beleuchtungsstrahlengang
- 58: Ausnehmung für Öffnung 48 für Beobachtungsstrahlengang
- 70: Lichtleiter
- 78: distales Ende des Lichtleiters 70
- 79: Lichtaustrittsfläche des Lichtleiters 70
- 80: Fensterbauteil im Beobachtungsstrahlengang
- 101: erster Schritt (Bereitstellen eines Rohres für das Mantelbauteil)
- 102: zweiter Schritt (Erzeugen des Mantelbauteils aus dem Rohr)
- 103: dritter Schritt (Bereitstellen eines Werkstücks)
- 104: vierter Schritt (Erzeugen einer Durchgangsöffnung im Werkstück)
- 105: fünfter Schritt (Erzeugen des Frontbauteils mittels Drahterodierens)
- 106: sechster Schritt (Heften des Frontbauteils an das Mantelbauteil)
- 107: siebter Schritt (Fügen des Frontbauteils an das Mantelbauteil)
- 108: achter Schritt (Einsetzen einer Lichtleiteinrichtung)
- 109: neunter Schritt (Erzeugen der Mantelfläche durch Abdrehen/Abschleifen)
- 110: zehnter Schritt (Erzeugen der Frontfläche durch Abschleifen und Polieren)
- 111: elfter Schritt (Bereitstellen eines Schaftrohres)
- 112: zwölfter Schritt (Fügen des distalen Endes an ein Schaftrohr)
- 123: dritter Schritt (Bereitstellen eines Blechs)
- 124: vierter Schritt (Erzeugen einer Durchgangsöffnung im Blech)
- 125: fünfter Schritt (plastisches Verformen des Blechs)

## Patentansprüche

1. **Endoskop** (10), mit:
einem **Außenschaft** (16) bestehend aus einen Schaftrohr (56) und einem separat gefertigten distalen Ende (20), dessen Oberfläche durch
eine Mantelfläche (23) und eine gekrümmte Frontfläche (24) gebildet wird,
wobei das **distale Ende** (20) des Außenschafts (16) an das den Außenschaft (16) überwiegend bildende Schaftrohr (56) gefügt ist und das distale Ende (20) ein Mantelbauteil (30) und ein gekrümmtes Frontbauteil (40), das an das Mantelbauteil (30) gefügt ist, und eine Kante (25) zwischen der Mantelfläche (23) und der Frontfläche (24) umfasst,
**dadurch gekennzeichnet, dass**
die Mantelfläche (23) von der Oberfläche des Mantelbauteils (30) und einer Randfläche (43) des Frontbauteils (40) gebildet wird und die Frontfläche (24) durch das Frontbauteil (40) gebildet wird;
wobei ein **Abstand** einer **Fügefläche** (39; 49) zwischen dem Mantelbauteil (30) und dem Frontbauteil (40) von der **Kante** (25) zwischen der Mantelfläche (23) und der gekrümmten Frontfläche (24) nicht größer als eine Wandstärke des Mantelbauteils (30) oder nicht größer als eine Wandstärke des Frontbauteils (40) ist,
wobei der Abstand in Richtung zur lokalen Flächennormale der Fügefläche gemessen wird,
wobei im Fall einer gebrochenen, abgerundeten oder gefasten Kante (25) zwischen Mantelfläche (23) und Frontfläche (24) mit dem Abstand zu dieser Kante (25) der Abstand zu der Linie gemeint ist, in der sich Mantelfläche (23) und Frontfläche (24) bei nicht gebrochener, abgerundeter oder gefaster Kante bzw. vor dem Brechen, Abrunden oder Fasen der Kante (25) schnitten,
wobei das **Frontbauteil** (40) von Öffnungen (47, 48) im Frontbauteil (40) abgesehen die **gesamte Frontfläche** (24) des distalen Endes (20) des Außenschafts (16) bildet.

2. Endoskop (10) nach dem vorangehenden Anspruch, bei dem das **Mantelbauteil** (30) höchstens von einem Randstreifen (43), dessen Breite durch die Wandstärke des Frontbauteils (40) bedingt ist, abgesehen die **gesamte Mantelfläche** (23) des distalen Endes (20) des Außenschafts (16) bildet.

3. Endoskop (10) nach Anspruch 1 oder 2, bei dem das **Frontbauteil** (40) von Öffnungen (47, 48) im Frontbauteil (40) und von einem Randstreifen (34), dessen Breite der Wandstärke des Mantelbauteils (30) entspricht, abgesehen die **gesamte Frontfläche** (24) des distalen Endes (20) des Außenschafts (16) bildet.

4. Endoskop (10) nach einem der Ansprüche 1, 2 oder 3, bei dem das Frontbauteil (40) eine Teilfläche (43) der Mantelfläche (23) und das Mantelbauteil (30) eine Teilfläche (34) der Frontfläche (24) des distalen Endes (20) des Außenschafts (16) bildet.

5. Endoskop (10) nach einem der vorangehenden Ansprüche, bei dem das Frontbauteil (40) aus einem plastisch verformten **Blech** (55) gebildet ist.

6. Verfahren zum Fertigen eines Endoskops (10) mit folgenden Schritten:
Bereitstellen (111) eines Schaftrohres (56);
**Fertigen eines distalen Endes** (20) eines Außenschafts (16) für ein Endoskop (10), wobei die Oberfläche des distalen Endes (20) des Außenschafts (16) durch eine Mantelfläche (23) und eine gekrümmte Frontfläche (24) gebildet wird, die in einer Kante (25) aneinandergrenzen, mit folgenden Schritten:
Bereitstellen (101, 102) eines Mantelbauteils (30);
Bereitstellen (103, 104, 105; 123; 124; 125) eines gekrümmten **Frontbauteils** (40);
wobei das Bereitstellen des Frontbauteils (40) folgenden Schritt umfasst:
Erzeugen (104; 124) einer **Durchgangsöffnung** (47, 48), die vorgesehen ist, um zumindest entweder eine Lichtaustrittsfläche (79) in einem Beleuchtungsstrahlengang (70) oder ein Fensterbauteil (80) in einem Beobachtungsstrahlengang aufzunehmen,
wobei die Mantelfläche (23) von der Oberfläche des Mantelbauteils (30) und einer Randfläche (43) des Frontbauteils (40) gebildet wird und die Frontfläche (24) durch das Frontbauteil (40) gebildet wird;
Fügen (106, 107) des Frontbauteils (40) an das Mantelbauteil (30) an einer **Fügefläche** (39; 49), deren Abstand von der Kante (25) zwischen Mantelfläche (23) und Frontfläche (24) beim fertiggestellten distalen Ende (20) nicht größer als eine Wandstärke des Mantelbauteils (30) oder nicht größer als eine Wandstärke des Frontbauteils (40) ist,
wobei der Abstand in Richtung zur lokalen Flächennormale der Fügefläche gemessen wird,
wobei im Fall einer gebrochenen, abgerundeten oder gefasten Kante (25) zwischen Mantelfläche (23) und Frontfläche (24) mit dem Abstand zu dieser Kante (25) der Abstand zu der Linie gemeint ist, in der sich Mantelfläche (23) und Frontfläche (24) bei nicht gebrochener, abgerundeter oder gefaster Kante bzw. vor dem Brechen, Abrunden oder Fasen der Kante (25) schnitten,
wobei das Frontbauteil (40) von Öffnungen (47, 48) im Frontbauteil (40) abgesehen die gesamte Frontfläche (24) des distalen Endes (20) des Außenschafts (16) bildet,
wobei das Mantelbauteil (30) separat gefertigt und anschließend an das Schaftrohr (56) gefügt (112) wird.

7. Verfahren nach dem vorangehenden Anspruch, bei dem das Bereitstellen des **Frontbauteils** (40) folgende Schritte umfasst:
Bereitstellen (103) eines **Werkstücks** (54);
Erzeugen (105) des Frontbauteils (40) aus dem Werkstück (54) zumindest entweder mittels **Erodierens** oder mittels **Laserschneidens.**

8. Verfahren nach Anspruch 6, bei dem das Bereitstellen des Frontbauteils (40) folgende Schritte umfasst:
Bereitstellen (123) eines **Blechs** (55);
plastisches Verformen (125) des Blechs (55).

9. Verfahren nach einem der Ansprüche 6 bis 8, bei dem das Fügen des Mantelbauteils (30) und des Frontbauteils (40) folgende Schritte umfasst:
**Heften** (106) des Frontbauteils (40) an das Mantelbauteil (30);
**Fügen** (107) des Frontbauteils (40) mit dem Mantelbauteil (30) entlang einer gesamten Fügefläche (39; 49) zwischen dem Mantelbauteil (30) und dem Frontbauteil (40).

## Claims

1. Endoscope (10), with:
an outer shank (16) composed of a shank tube (56) and of a separately produced distal end (20) whose surface is formed by a jacket face (23) and a curved front face (24),
wherein the distal end (20) of the outer shank (16) is joined to the shank tube (56) forming most of the outer shank (16), and the distal end (20) comprises a jacket component (30) and a curved front component (40), which is joined to the jacket component (30), and an edge (25) between the jacket face (23) and the front face (24),
**characterized in that**
the jacket face (23) is formed by the surface of the jacket component (30) and an edge face (43) of the front component (40), and the front face (24) is formed by the front component (40);
wherein a distance of an interface (39; 49) between the jacket component (30) and the front component (40) from the edge (25) between the jacket face (23) and the curved front face (24) is no greater than a wall thickness of the jacket component (30) or no greater than a wall thickness of the front component (40),
wherein the distance is measured in the direction of the local surface normal of the interface,
wherein, in the case of a broken, rounded or bevelled edge (25) between jacket face (23) and front face (24), the distance to this edge (25) is understood to mean the distance to the line in which jacket face (23) and front face (24) intersect when the edge is not broken, rounded or bevelled, or before the edge (25) is broken, rounded or bevelled,
wherein the front component (40) forms the entire front face (24) of the distal end (20) of the outer shank (16), except for openings (47, 48) in the front component (40).

2. Endoscope (10) according to the preceding claim, in which the jacket component (30) forms the entire jacket face (23) of the distal end (20) of the outer shank (16), except at most for an edge strip (43) whose width is determined by the wall thickness of the front component (40).

3. Endoscope (10) according to Claim 1 or 2, in which the front component (40) forms the entire front face (24) of the distal end (20) of the outer shank (16), except for openings (47, 48) in the front component (40) and except for an edge strip (34) whose width corresponds to the wall thickness of the jacket component (30).

4. Endoscope (10) according to one of Claims 1, 2 and 3, in which the front component (40) forms a partial area (43) of the jacket face (23) and the jacket component (30) forms a partial area (34) of the front face (24) of the distal end (20) of the outer shank (16).

5. Endoscope (10) according to one of the preceding claims, in which the front component (40) is formed from a plastically deformed metal sheet (55).

6. Method for producing an endoscope (10), with the following steps:
providing (111) a shank tube (56);
producing a distal end (20) of an outer shank (16) for an endoscope (10), wherein the surface of the distal end (20) of the outer shank (16) is formed by a jacket face (23) and a curved front face (24) that border each other in an edge (25), with the following steps:
providing (101, 102) a jacket component (30);
providing (103, 104, 105; 123; 124; 125) a curved front component (40);
wherein the provision of the front component (40) comprises the following step:
generating (104; 124) a through-opening (47, 48), which is intended at least to receive either a light outlet surface (79) in an illumination beam path (70) or a window component (80) in an observation beam path,
wherein the jacket face (23) is formed by the surface of the jacket component (30) and an edge face (43) of the front component (40), and the front face (24) is formed by the front component (40);
joining (106, 107) the front component (40) to the jacket component (30) at an interface (39; 49) whose distance from the edge (25) between jacket face (23) and front face (24) is no greater than a wall thickness of the jacket component (30) or no greater than a wall thickness of the front component (40) once the distal end (20) has been finished,
wherein the distance is measured in the direction of the local surface normal of the interface,
wherein, in the case of a broken, rounded or bevelled edge (25) between jacket face (23) and front face (24), the distance to this edge (25) is understood to mean the distance to the line in which jacket face (23) and front face (24) intersect when the edge is not broken, rounded or bevelled, or before the edge (25) is broken, rounded or bevelled,
wherein the front component (40) forms the entire front face (24) of the distal end (20) of the outer shank (16), except for openings (47, 48) in the front component (40),
wherein the jacket component (30) is produced separately and then joined (112) to the shank tube (56).

7. Method according to the preceding claim, in which the provision of the front component (40) comprises the following steps:
providing (103) a workpiece (54);
generating (105) the front component (40) from the workpiece (54) at least either by means of erosion or by means of laser cutting.

8. Method according to Claim 6, in which the provision of the front component (40) comprises the following steps:
providing (123) a metal sheet (55);
plastically deforming (125) the metal sheet (55).

9. Method according to one of Claims 6 to 8, in which the joining of the jacket component (30) and of the front component (40) comprises the following steps:
placing (106) the front component (40) onto the jacket component (30);
joining (107) the front component (40) to the jacket component (30) along an entire interface (39; 49) between the jacket component (30) and the front component (40).

## Revendications

1. Endoscope (10), avec:
une tige extérieure (16) se composant d'un tube de tige (56) et d'une extrémité distale (20) fabriquée séparément, dont la surface est formée par une face latérale (23) et une face frontale courbe (24),
dans lequel l'extrémité distale (20) de la tige extérieure (16) est jointe au tube de tige (56) formant majoritairement la tige extérieure (16) et l'extrémité distale (20) comprend un composant latéral (30) et un composant frontal courbe (40), qui est joint au composant latéral (30), et une arête (25) entre la face latérale (23) et la face frontale (24),
**caractérisé en ce que**
la face latérale (23) est formée par la surface du composant latéral (30) et une face périphérique (43) du composant frontal (40) et la face frontale (24) est formée par le composant frontal (40);
dans lequel une distance d'une face de jonction (39; 49) entre le composant latéral (30) et le composant frontal (40) à partir de l'arête (25) entre la face latérale (23) et la face frontale courbe (24) n'est pas plus grande qu'une épaisseur de paroi du composant latéral (30) ou n'est pas plus grande qu'une épaisseur de paroi du composant frontal (40),
dans lequel la distance est mesurée dans la direction de la normale locale à la surface de la face de jonction,
dans lequel, dans le cas d'une arête brisée, arrondie ou chanfreinée (25) entre la face latérale (23) et la face frontale (24), on entend par la distance jusqu'à cette arête (25) la distance jusqu'à la ligne suivant laquelle la face latérale (23) et la face frontale (24) se coupent lorsque l'arête n'est pas brisée, arrondie ou chanfreinée ou avant que l'arête (25) soit brisée, arrondie ou chanfreinée,
dans lequel le composant frontal (40) forme, hormis des ouverture (47, 48) dans le composant frontal (70), toute la face frontale (24) de l'extrémité distale (20) de la tige extérieure (16).

2. Endoscope (10) selon la revendication précédente, dans lequel le composant latéral (30) forme, hormis au maximum une bande périphérique (43) dont la largeur est déterminée par l'épaisseur de paroi du composant frontal (40), toute la face latérale (23) de l'extrémité distale (20) de la tige extérieure (16).

3. Endoscope (10) selon la revendication 1 ou 2, dans lequel le composant frontal (40) forme, hormis des ouvertures (47, 48) dans le composant frontal (40) et une bande périphérique (34), dont la largeur correspond à l'épaisseur de paroi du composant latéral (30), toute la face frontale (24) de l'extrémité distale (20) de la tige extérieure (16).

4. Endoscope (10) selon l'une des revendications 1, 2 ou 3, dans lequel le composant frontal (40) forme une face partielle (43) de la face latérale (23) et le composant latéral (30) forme une face partielle (34) de la face frontale (24) de l'extrémité distale (20) de la tige extérieure (16).

5. Endoscope (10) selon l'une quelconque des revendications précédentes, dans lequel le composant frontal (40) est formé d'une tôle (55) déformée plastiquement.

6. Procédé de fabrication d'un endoscope (10) comportant les étapes suivantes:
préparer (111) un tube de tige (56);
fabriquer une extrémité distale (20) d'une tige extérieure (16) pour un endoscope (10), dans lequel la surface de l'extrémité distale (20) de la tige extérieure (16) est formée par une face latérale (23) et une face frontale courbe (24), qui se rejoignent sur une arête (25), avec les étapes suivantes:
préparer (101, 102) un composant latéral (30);
préparer (103, 104, 105; 123, 124, 125) un composant frontal courbe (40);
dans lequel la préparation du composant frontal (40) comprend les étapes suivantes:
produire (104; 124) une ouverture de passage (47, 48), qui est prévue pour recevoir au moins soit une face de sortie de lumière (79) dans un chemin de rayons d'éclairage (70) soit un composant de fenêtre (80) dans un passage de rayons d'observation,
dans lequel la face latérale (23) est formée par la surface du composant latéral (30) et une face périphérique (43) du composant frontal (40) et la face frontale (24) est formée par le composant frontal (40);
joindre (106, 107) le composant frontal (40) au composant latéral (30) dans une face de jonction (39; 49), dont la distance à partir de l'arête (25) entre la face latérale (23) et la face frontale (24) pour l'extrémité distale terminée (20) n'est pas plus grande qu'une épaisseur de paroi du composant latéral (30) ou n'est pas plus grande qu'une épaisseur de paroi du composant frontal (40),
dans lequel la distance est mesurée dans la direction de la normale locale à la face de jonction,
dans lequel, dans le cas d'une arête brisée, arrondie ou chanfreinée (25), entre la face latérale (23) et la face frontale (24), on entend par la distance jusqu'à cette arête (25) la distance jusqu'à une ligne suivant laquelle la face latérale (23) et la face frontale (24) se coupent lorsque l'arête n'est pas brisée, arrondie ou chanfreinée ou avant que l'arête (25) soit brisée, arrondie ou chanfreinée,
dans lequel le composant frontal (40) forme, hormis des ouvertures (47, 48) dans le composant frontal (40), toute la face frontale (24) de l'extrémité distale (20) de la tige extérieure (16),
dans lequel le composant latéral (30) est fabriqué séparément et est ensuite joint (112) au tube de tige (56).

7. Procédé selon la revendication précédente, dans lequel la préparation du composant frontal (40) comprend les étapes suivantes:
préparer (103) une pièce (54);
produire (105) le composant frontal (40) à partir de la pièce (54) au moins soit par érosion soit par coupe au laser.

8. Procédé selon la revendication 6, dans lequel la préparation du composant frontal (40) comprend les étapes suivantes:
préparer (123) une tôle (55);
déformer plastiquement (125) la tôle (55).

9. Procédé selon l'une quelconque des revendications 6 à 8, dans lequel la jonction du composant latéral (30) et du composant frontal (40) comprend les étapes suivantes:
agrafer (106) le composant frontal (40) au composant latéral (30);
joindre (107) le composant frontal (40) au composant latéral (30) le long d'une face de jonction totale (39; 49) entre le composant latéral (30) et le composant frontal (40).
